# EUROPEAN PATENT SPECIFICATION

(11) **EP 4 042 953 B1**
(45) Date of publication and mention of the grant of the patent: **11.11.2026**
(21) Application number: 22166858.5
(22) Date of filing: 08.07.2016
(51) Int. Cl.: A61B 17/16, A61B 17/00

(54) **EXTERNAL HAND CONTROL FOR SURGICAL POWER TOOL**
EXTERNE HANDSTEUERUNGSVORRICHTUNG FÜR CHIRURGISCHES ELEKTROWERKZEUG
COMMANDE DE POIGNÉE EXTERNE POUR OUTIL ÉLECTRIQUE CHIRURGICAL

(30) Priority: 09.07.2015 US 201514795203
(43) Date of publication of application: 17.08.2022
(62) Document number(s) of the earlier application(s) in accordance with Art. 76 EPC: 20159337.3 / 3 679 872; 16178569.6 / 3 132 757
(73) Proprietor: DePuy Synthes Products, Inc., Raynham, MA 02767 (US)
(72) Inventor: BUCINA, Stephen M., Palm Beach Gardens, FL 33410 (US); KASPER, James H., Palm Beach Gardens, FL 33410 (US); BEAUSSE, Cedric, Raynham, MA 02767 (US); NOWAK, Piotr, Palm Beach Gardens, FL 33410 (US); NARDUCCI, David S., Palm Beach Gardens, FL 33410 (US); NEICONI, Ovidiu, Palm Beach Gardens, FL 33410 (US)
(74) Representative: Carpmaels & Ransford LLP

(56) References cited:
- US-A- 5 373 317
- US-A- 5 873 814
- US-A1- 2005 020 909
- US-A1- 2008 077 158
- US-A1- 2009 253 472
- US-A1- 2013 307 529

## Description

### TECHNICAL FIELD

This invention relates to a device for the controlled operation of a surgical power tool, and more particularly to a wireless control device mounted to the handpiece of the power tool.

### BACKGROUND

Many surgical procedures require the use of electrically or pneumatically powered tools, such as burrs and saws. Typically, operation of a surgical power tool is controlled either by a foot pedal or a hand control/input (e.g., a pushbutton, switch, or lever located on the handpiece of the tool). A common drawback of a foot pedal controller is the lack of mobility in the operating room. Many surgical suites are crowded with medical equipment and hospital staff, making the physician's movement around the patient (and relocation of the foot pedal) complicated and time consuming. A system utilizing a controller located along the handpiece of the power tool can cause problems of visibility of control of the power tool. For example, depending on the surgical procedure, patient anatomy, and surgeon's hand size and grip position, visibility of the surgical site and control of the power tool can be limited/compromised by the surgeon's hand or the controller itself. Accordingly, there remains a need in the art to provide a safe and effective apparatus and method for ensuring visibility of a surgical site and superior physician control of a hand-operated power tool.

US 2005/020909 discloses a display unit for use with a surgical navigation system including a body, a screen capable of displaying data, and an attachment device to attach the display unit to a body of a surgical instrument to be manipulated relative to a patient's anatomy. Furthermore, US 2008/0077158 discloses a surgical tool comprising an onboard screen which could be a touch screen for allowing input controls directly through the onboard screen.

### SUMMARY

In a first aspect, the invention provides for a control device for a surgical power tool. The control device for a surgical power tool comprises a housing sized and configured to couple to a surgical power tool, the housing comprising a top housing and a bottom housing, wherein the top housing and the bottom housing are coupled together; an input element, the input element comprising a base portion and an input surface provided at the housing for receiving a user input, the input surface further comprising an anchor which extends from the base portion in a direction away from the input surface, the anchor configured to couple the input element to the bottom housing; and a control unit within the housing for sending user input information received at the input surface to the surgical power tool, the control unit comprising an opening, where the anchor is configured to extend from the base portion of the input surface and through the opening provided in the control unit, to couple the input element to the bottom housing.

Another aspect of the present invention is directed to a surgical power tool and an associated control device. The control device can be coupled to the surgical power tool, the control device can be moved along or around the handpiece of the surgical power tool. The control device can include a housing, a pressure responsive touch pad, and a control unit. The housing can include arms for coupling the control device to the power tool. The arms can extend from the housing and can engage at least a portion of the outer perimeter of the handpiece of the power tool. The pressure responsive touch pad can be located on a top surface of the control device and can receive a user input. The control unit can send user input information received at the input element to the power tool for directing a speed of the power tool or other functions.

Additionally, a method of controlling a surgical power tool using a control unit movable and releasably coupled to the handpiece of a surgical power tool is discussed.

The control device can be first oriented with respect to the handpiece such that the opening between arms extending from the bottom surface of the control device is positioned over the handpiece. The control device can then be pressed onto the handpiece as the arms flex/expand to advance over and/or around the handpiece. Once coupled to the handpiece, the control device can be moved by the surgeon along the body of the handpiece. The surgeon can slide and/or rotate the control device along/around the handpiece. The surgeon can then activate the power tool by providing pressure at the input surface. By varying the pressure at the input surface, the input information can be varied resulting in a corresponding change in the operation of the power tool.

The details of one or more embodiments of the invention are set forth in the accompanying drawings and the description below. Other features, objects, and advantages of the invention will be apparent from the description and drawings, and from the claims.

### DESCRIPTION OF DRAWINGS

The device is explained in greater detail in the following drawings. The drawings are merely exemplary to illustrate the structure of preferred devices and certain features that may be used singularly or in combination with other features. The invention should not be limited to the examples shown.
FIGS. 1-2 are perspective views of an example control device;
FIGS. 3 is a perspective view of an example control device and surgical power tool;
FIGS. 4A-4B are perspectives views of an example control device coupled to a surgical power tool;
FIG. 5 is an exploded view of the example control device of FIG. 1;
FIG. 6 is a section view of the example control device of FIG. 1;
FIGS. 7A-7B are perspective views of an example top housing cover;
FIG. 7C is a top view of the top housing cover of FIG. 7A;
FIG. 7D is a side view of the top housing cover of FIG. 7A;
FIG. 7E is a bottom view of the top housing cover of FIG. 7A;
FIGS. 8A-8B are perspective views of an example bottom housing cover;
FIG. 8C is a top view of the bottom housing cover of FIG. 8A;
FIG. 8D is a side view of the bottom housing cover of FIG. 8A;
FIG. 8E is a bottom view of the bottom housing cover of FIG. 8A;
FIG. 8F is an end view of the bottom housing cover of FIG. 8A;
FIGS. 9A-9B are perspective views of an example input element;
FIG. 9C is a top view of the example input element of FIG. 9A;
FIG. 9D is a side view of the example input element of FIG. 9A;
FIG. 9E is a bottom view of the example input element of FIG. 9A;
FIG. 9F is an end view of the example input element of FIG. 9A;
FIG. 10A is a perspective view of an example control unit;
FIG. 10B is a top view of the example control unit of FIG. 10A;
FIGS. 11A-11B are perspective views of an example slide cover;
FIG. 11C is a top view of the example slide cover of FIG. 11A;
FIG. 11D is a side view of the example slide cover of FIG. 11A;
FIG. 11E is a bottom view of the example slide cover of FIG. 11A;
FIG. 11F is an end view of the example slide cover of FIG. 11A;
FIG. 12 is a perspective views of an example control device;
FIG. 13 is a perspective views of an example control device;
FIG. 14 is a perspective views of an example control device; and
FIG. 15 is a perspective views of an example control device.

Like reference symbols in the various drawings indicate like elements.

### DETAILED DESCRIPTION

Certain terminology is used in the following description for convenience only and is not limiting. The words "right," "left," "lower," and "upper" designate direction in the drawings to which reference is made. The words "inner" and "outer" refer to directions toward and away from, respectively, the geometric center of the described feature or device. The words "distal" and "proximal" refer to directions taken in context of the item described and, with regard to the instruments herein described, are typically based on the perspective of the surgeon using such instruments. The terminology includes the above-listed words, derivatives thereof, and words of similar import.

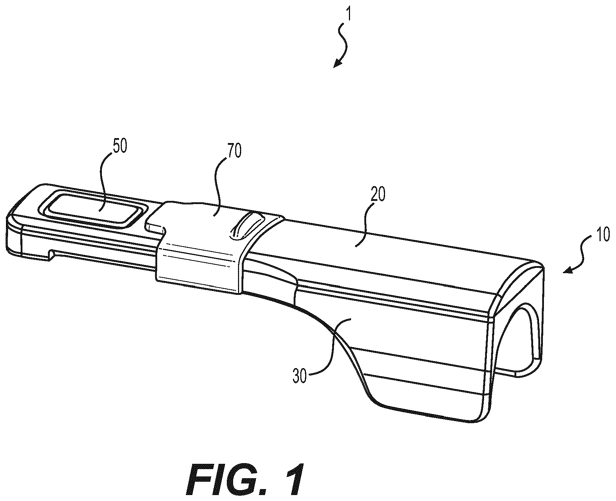

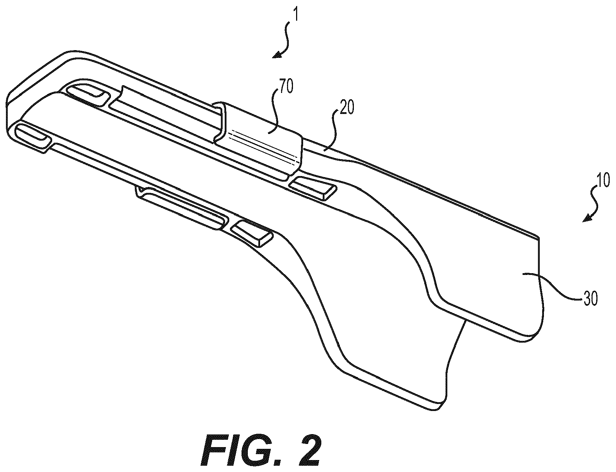

Certain examples of the invention will now be described with reference to the drawings. In general, such embodiments relate to a control device movably and releasably coupled to the handpiece of a surgical power tool for use in controlling the operation of the power tool. FIG. 1 provides a top perspective view of an example control device 1. The control device 1 can include a housing 10, an input element 50, and a control unit 60 (not shown). The housing 10 is sized and configured for coupling the control device 1 to a surgical power tool 100. As will be explained in more detail below, the housing 10 can include various engagement features that allow the housing 10/control device 1 to be releasably and/or moveably coupled to the power tool 100. For example, as illustrated in FIG. 2, the bottom surface of the housing 10 can have a shape complementary to the outer surface of the power tool 100. Additionally, the housing 10 can include arms 32 for securing the housing 10 to the power tool 100.

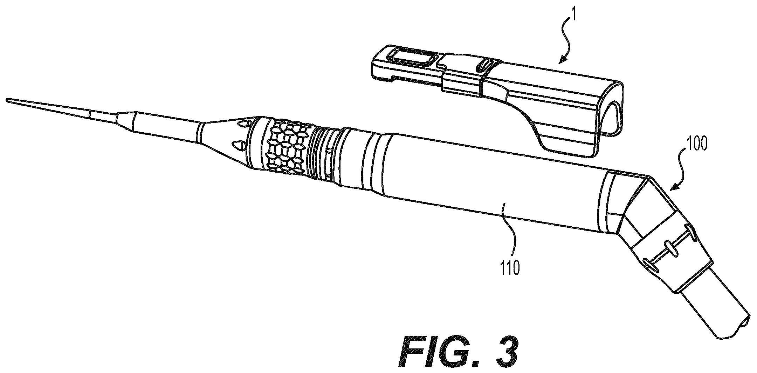

Figure 3 illustrates the control device 1 aligned with the handpiece 110 of an example surgical power tool 100. It is contemplated that the example power tool 100 can include any number of power tools typically used in a surgical procedure. The power tool 100 can be used in any number of surgical procedures in orthopaedics, traumatology, neurosurgery, neurotology, spinal procedures, otolaryngology, cranio-facial surgery, etc. Example power tools 100 include, for example, drills, saws, shavers, drivers, reamers, pinners, staplers, microdebriders, etc. For example, the Anspach XMax^{®} series or the E-Max^{®} series, pneumatic and electric power tools, respectively, are contemplated for use with the disclosed control device and are commercially available from DePuy Synthes Products, Inc. of Raynham, Massachusetts.

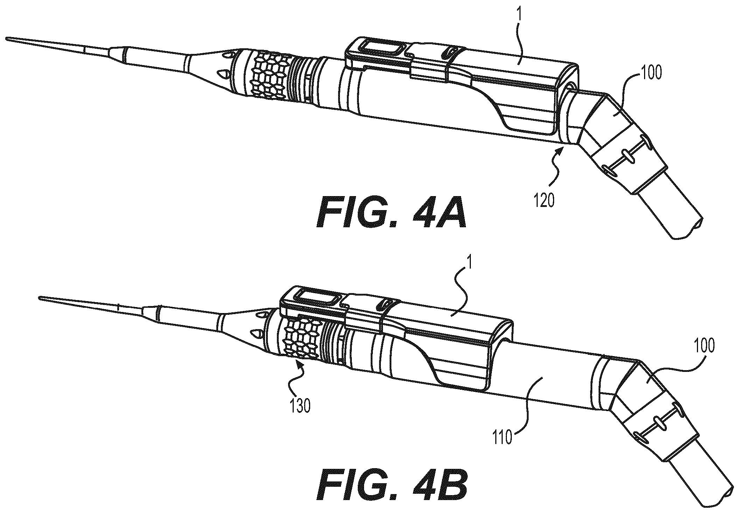

The power tool 100 can be electrically (wired or wireless) and/or pneumatically powered. As will be explained in more detail below, the housing 10 of the control device 1 can be coupled to the handpiece 110 of the power tool 100. Depending on the surgical procedure, patient anatomy, and surgeon grip position and visibility, the surgeon may wish to position the control device 1 at various locations along and/or around the handpiece 110 of the power tool 100. For example, as different length attachments (e.g., burrs, blades) are attached to the handpiece 110, the distance between the surgical site/working area and the surgeon's grip position on the control device 1/handpiece 110 increases, making control more difficult. The surgeon may wish to position the control device 1 forward on the handpiece, closer to the surgical site/working area, thereby giving the surgeon more control and stability over the power tool 100/handpiece 110. Accordingly, it is contemplated that the control device 1 can be positioned at any location along the handpiece 110. For example, as illustrated in FIG. 4A, the control device 1 can be located rearward on the handpiece 110, proximate the control end 120 of the power tool 100. As illustrated in FIG. 4B, the control device 1 can be located forward on the handpiece 110, proximate the operating end 130 of the power tool 100.

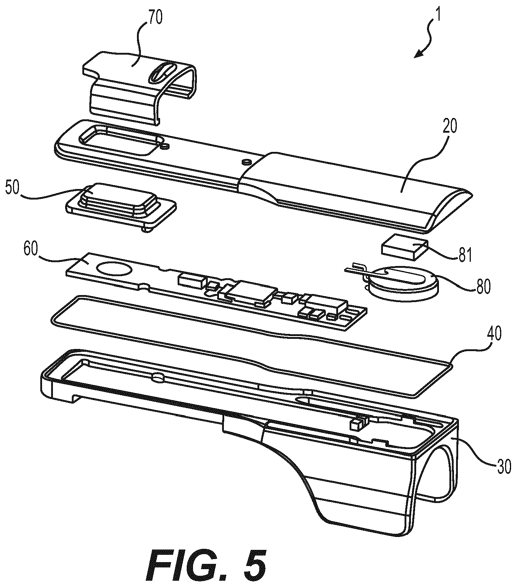

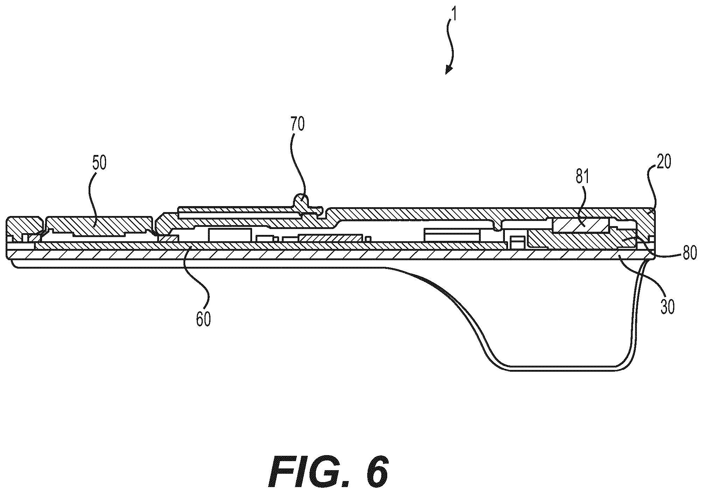

FIG. 5 provides an exploded view of the various components of an example control device 1. FIG. 6 provides a cross-section view of the example control device 1 illustrated in FIG. 1. The control device 1 can include a housing 10, an input element 50, a control unit 60, and a slide cover 70 movable along the housing 10. As illustrated in FIGS. 5 and 6, the housing 10 can include a top housing 20 and a bottom housing 30. The top housing 20 and the bottom housing 30 can be permanently or releasably coupled together. The top housing 20 and the bottom housing 30 can be coupled using screws, hooks, clips, welds, or any other form of mechanical fastener known in the art. It is also contemplated that the top housing 20 and the bottom housing 30 can be coupled using a chemical fastener/adhesive. For example, as illustrated in FIG. 5, the top housing and the bottom housing 30 can be coupled using a pressure-sensitive adhesive 40 or an epoxy. It is contemplated that the top housing 20 and the bottom housing 30 can be formed as a single, integral housing.

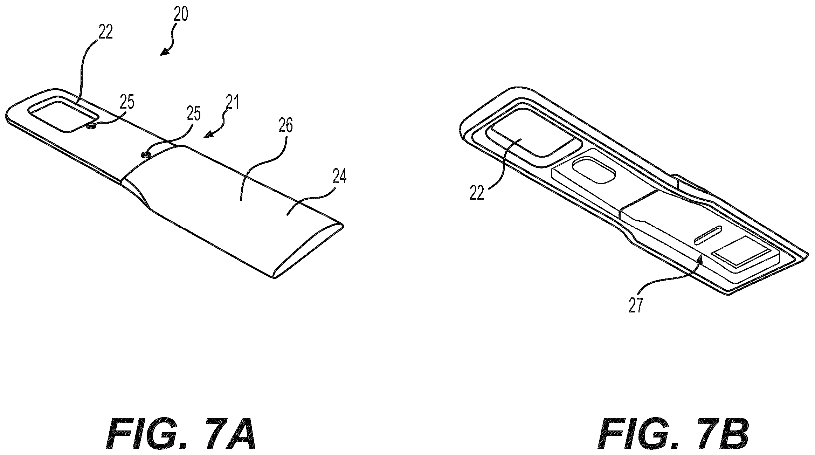

FIGS. 7A-7E provide various views of an example top housing 20. The top housing 20 can include an elongated body portion 21 sized and configured to extend along a length of the handpiece 110 of the surgical power tool 100. The top housing 20 can include an opening 22 providing access to the input element 50. As will be explained in more detail below, the opening 22 can be located proximate an input position (input element contacts 63) on the control unit 60 such that user input information received at the input element 50 is transferred/received by the control unit 60. The top housing 20 can also include a recessed surface 23 sized and configured to accommodate movement of the slide cover 70. The top housing 20 can also an engagement feature for fixing the position of the slide cover 70. For example, the top surface 24 of the top housing 20 can include protrusions 25 for engaging a corresponding recess 73 provided in the slide cover 70. The first protrusion 25A can secure the slide cover 70 is a first, non-operating, position where the slide cover 70 covers over at least a portion of the input element 50 and prevents accidental activation of the power tool 100. The second protrusion 25B can be used to secure the slide cover 70 in a second, operating, position away from the input element 50, illustrated, for example, in FIG. 1.

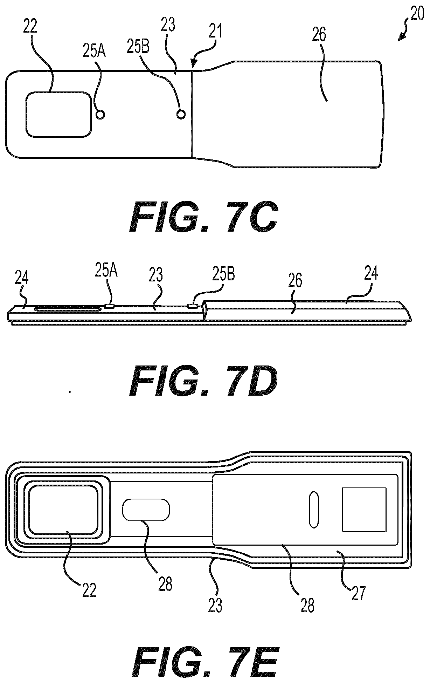

The top housing 20 can include an outer grip surface 26 to accommodate a secure grip by the surgeon during the use, illustrated in FIGS. 7A, 7C, and 7D. The grip surface 26 can include a contoured outer surface and/or textured surface features to enhance grip/purchase between the surgeon's hand and the control device 1. The inside surface 27 of the top housing 20 can include recessed portions 28 as illustrated in FIGS. 7B and 7E. These recessed portions 28 can be sized and configured to accommodate the input element 50 and control unit 60 components.

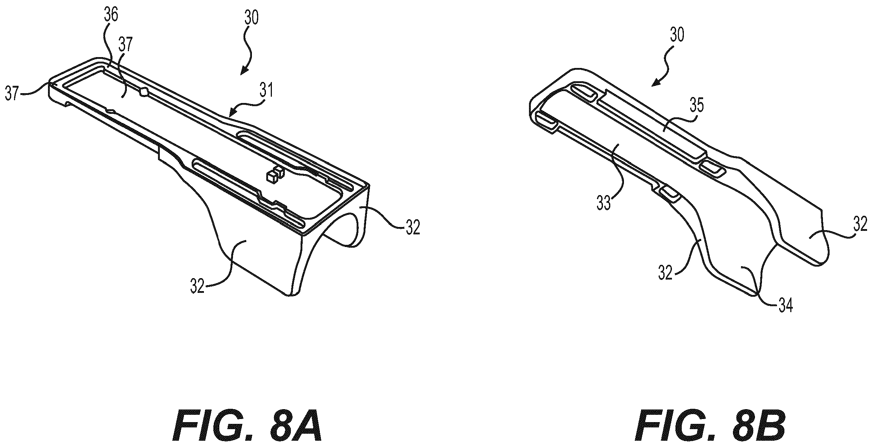

FIGS. 8A-8F provide various views of an example bottom housing 30. The bottom housing 30 can include an elongated body portion 31 sized and configured to extend along a length of the handpiece 110 of the surgical power tool 100. The bottom housing 30 can include an engagement feature for coupling the bottom housing 30 to the handpiece 110. The bottom housing 30 can be permanently or releasably coupled to the handpiece 110. The engagement features can be provided such that the bottom housing 30 is moveably coupled along the handpiece 110. For example, the bottom housing 30 may slide along and/or rotate around the handpiece 110. The surgeon may couple the housing 10/bottom housing 30 to the handpiece 110 and wish to relocate along the body of the handpiece 110 before/during/after the surgical procedure. In which case, the housing 10/bottom housing 30 may slide along the body of the handpiece 110 to the desired position. The housing 10/bottom housing 30 may also be fixed to the power tool 100 such that it does not side along/rotate with respect to the body of the handpiece 110.

The engagement features included on the bottom housing 30 can include arms 32 extending in a direction away from bottom surface 33 of the housing 10/bottom housing 30 and around/towards the handpiece 110 of the power tool 100. As illustrated in FIGS. 4A and 4B, the arms 32 can be configured to engage at least a portion of the outer perimeter of the handpiece 110. It is contemplated that the arms 32 can be sized and configured to engage a portion of the power tool 100 other than the handpiece 110. The arms 32 can engage the outer perimeter of the handpiece 110/power tool 100 by a snap fit, press fit, a weld, or any other mechanical or chemical fastener known in the art.

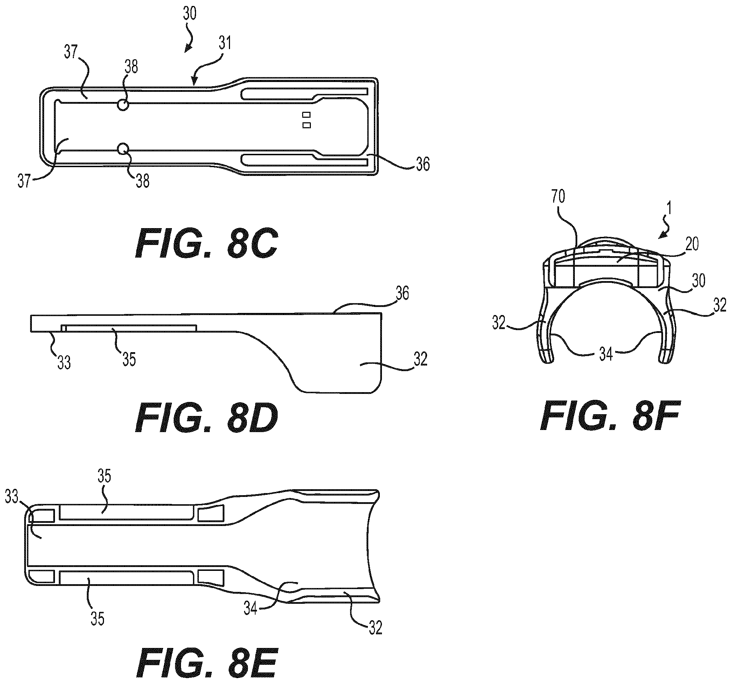

The arms 32 can include an inside surface 34 having a shape corresponding to the shape of the handpiece 110/power tool. As illustrated in FIGS. 8B, 8E and 8F, the inside surface 34 defines a round surface corresponding to the outside perimeter of the handpiece 110. Likewise, the bottom housing 30 can include a bottom surface 33 having a shape corresponding to the shape of the handpiece 110/power tool 100. For example, as illustrated in FIGS. 8B and 8F, the bottom surface 33 has a rounded shaped corresponding to the cylindrical shape of the handpiece 110.

The bottom housing 30 can include recessed surfaces 35 sized and configured to accommodate movement of the slide cover 70. For example, as illustrated in FIGS. 2, 8A, 8B, 8D and 8E the bottom surface 33 of the bottom housing 30 can include recessed surfaces 35 extending along the left and right side of the body portion 31 of the bottom housing 30. The recessed surfaces 35 provide a groove/slot for the arms 72 of the slide cover 70. The recessed surfaces 35 can extend along the entire length of the bottom housing 30. Alternatively, as illustrated in FIGS. 8D and 8E, the recessed surfaces 35 can extend along only a portion of the length of the bottom housing 30 and the ends of the recessed surfaces 35 act as stops for the slide cover 70. That is, movement of the recessed cover between a first position and a second position is limited by contact between the arms 72 and the ends of the recessed surfaces 35.

The top surface 36 of the bottom housing 30 can include recessed portions 37 as illustrated in FIGS. 8A and 8C. These recessed portions 37 can be sized and configured to accommodate the input element 50 and the control unit 60. The inside surface 36 can also include an anchor point 38 for coupling the bottom housing 30 to the input element 50. For example, as illustrated in FIGS. 8A and 8C, the bottom housing 30 can include two circularshaped cavities 38 for receiving a similarly-shaped anchors 54 extending from the input element 50. The anchors 54 of the input element 50 can be permanently and/or releasably coupled to the bottom housing 30. The anchors 54 can be press fit, snap fit, welded, or coupled to the bottom housing 30 using any other mechanical or chemical fastener known in the art.

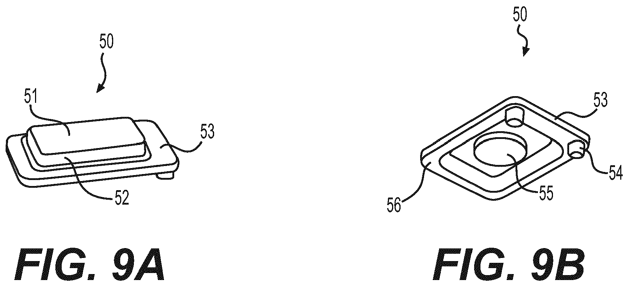

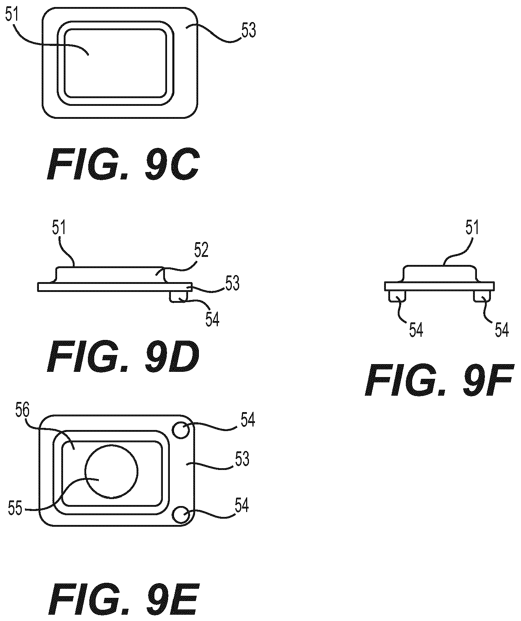

FIGS. 9A-9F provide various views of an example input element 50. The input element 50 can include a pressure responsive touch pad for receiving the surgeon's input. As will be explained in more detail below, the input element 50 can facilitate an electrical and/or mechanical connection that translates a user input at the input surface 51 to the control unit 60, thereby controlling operation of the surgical power tool 100.

The input surface 51 can be located at the top surface 24 of the top housing 10. The input surface 51 can be accessible via the opening 22 provided in the top housing 10. For example, as illustrated in FIGS. 1, 5, and 6, the input element 50 can extend from inside the housing 10 and partially into and/or through the opening 22 provided in the top housing 20. In an example control device 1, the input surface 51 is recessed below the top surface 24 of the top housing 20. The input surface 51 can have a shape corresponding to the shape of the opening 22. As illustrated in FIGS. 7C and 9C, the input surface 51 and the opening 22 can define rectangular shape. It is contemplated that the input surface 51 and/or the opening 22 can define can define any suitable shape including, for example, circular, elliptical, square, rectangular, or any other regular or irregular shape.

As outlined above, the input element 50 can include a pressure responsive touch pad. The input element 50 can be constructed from a flexible/compliant material. For example the input element 50 can be constructed from silicone rubber any other flexible/compliant material known in the art. In an example control device 1, the input element 50 can be constructed having uniform flexibility/elasticity. In another example, various portions of the input element 50 can have differing flexibility/elasticity. As illustrated in FIGS. 9A and 9D, the input surface 51 can extend in a direction parallel to the base portion of the input element 50. The input element 50 can include flexible side walls 52 that extend between the input surface 51 and the base portion 53. The flexible side walls 52 can be provided such that pressure on the input surface 51 can result in translational movement of the input surface 51 with respect to the base portion 53. The side walls 52 can have increased flexibility with respect to the input surface 51 and/or the base portion 53.

The input element 50 can be permanently or releasably coupled to the housing 10. As described above, and as illustrated in FIGS. 9B and 9D, the input element 50 includes anchors 54 sized and configured for coupling the input element 50 to the housing 10. The anchors 54 extend from the base portion 53 in a direction away from the input surface 51. The input element 50 can include any number of anchors 54 suitable for coupling the input element 50 to the housing 10. When assembled, the anchors 54 extend through opening 64 provided in the control unit 60 before coupling to the housing 10.

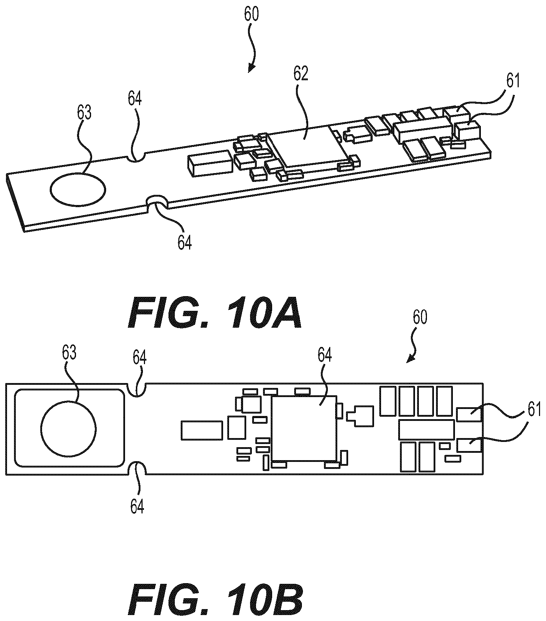

FIGS. 10A and 10B provide a perspective and plan view of an example control unit 60. The control unit 60 can be located within the housing 10 and can be used to send user input information received at the input element 50 to the surgical power tool 100 to direct operation of the power tool 100. For example, the control unit 60 can provide a wired or wireless communication link with the corresponding control unit of the power tool 100. The control unit 60 can include a PCB (printed circuit board), a flexible circuit or any other circuitry for electrically connecting the electronic components of the control unit 60 and providing two-way communication between the control unit 60 and the power tool 100. The control unit 60 can facilitate two-way communication between the control device 1 and the power tool 100, i.e., the control unit 60 be used to communicate input information received at the input element 50 to surgical power tool 100 and also receive operation information from the power tool 100.

Components of the control unit 60 can include battery contacts 61, a wireless communication unit 62 (e.g., radio chip antenna, microcontroller with integrated radio, etc.) for communicating with the power tool, and input element contacts 63, an analog-to-digital converter for converting the user's input into an electrical signal for wireless transmission to the power tool, etc. A battery 80 can be positioned in-line with the control unit 60. As illustrated in FIGS. 7E and 8C, the top housing 20 and the bottom housing 30 can include recessed portions 28 and 27 sized and configured to receive the battery to ensure the low profile design of the housing 10. A compressive pad 81 may be included within the housing 10 to secure the position of the battery 80 against contacts 61 provided on the control unit 60.

The input element 50 can be located within the control device 1/housing 10 at a position proximate the control unit 60 such that a user input at the input surface 51 activates the input element contacts 63 on the control unit 60. In an example control device 1, the input element 50 can include a carbon or gold pill 55 placed on the bottom surface 56 of the input element 50 proximate the contacts 63. The carbon pill 55 can be provided within the input element 50 by means of a silicone overmold (or other insulative material). Upon depression of the input element 50, the pill 55 comes into contact with the control unit contacts 63 and completes an electrical connection. As pressure is removed from the input surface 51, the electrical connection ends and the input element 50 and pill 55 return to their neutral position. The force to operate (actuation force) the input element 50 is approximately 100g (+/-25g).

Varying the user input provided at the input surface 51 can vary the input information received at/measured by the control unit 60. That is, varying the input information can result in a corresponding change in power tool 100 operation. For example, it is contemplated that the control device 1 can be used to control the on/off function, speed, torque, direction, illumination, irrigation, suction, or any other function completed by a surgical power tool. In one example, the increased pressure/force applied at the input surface 51 can correspond to an increased power tool 100 speed, torque, etc. Likewise, varying the location and/or direction of the user input on the input surface 51 can result in a corresponding change in input information and power tool 100 operation. For example, applying input force at a certain location on the input surface 51 can be used to provide a specific operation instructions to the power tool 100 (e.g., on/off, speed, torque, direction). Likewise, sweeping/moving the finger across the input surface 51 can also be used to provide a specific instruction to the power tool 100 (e.g., on/off, speed, torque, direction).

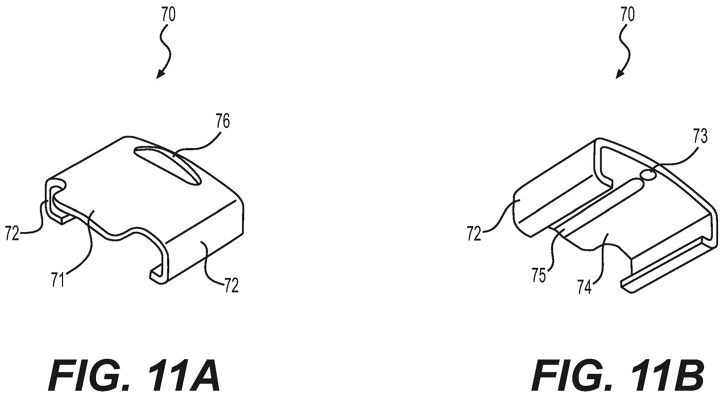

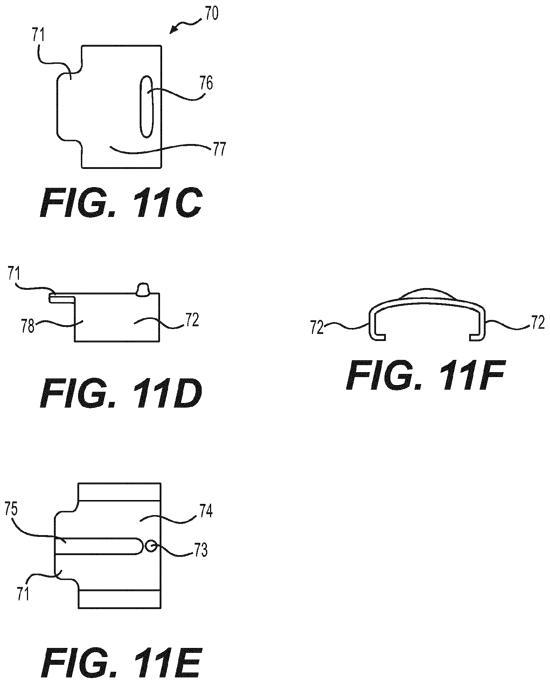

FIGS. 11A-11F provide various views of an example slide cover 70. The slide cover 70 is movable along the housing 10 (top housing 20 and bottom housing 30) between a first, non-operating, position where the slide cover 70 covers over at least a portion of the input element 50 and a second, operating, position allowing access to the input element 50. The second, operating, position is illustrated in FIG. 1. The slide cover 70 can include an extension 71 provided at the distal end of the slide cover 70 for extending over/covering the input surface 51 of the input element 50 when the slide cover 70 is in (or proximate) the first, non-operating, position.

The slide cover 70 can also include two downwardly extending arms 72 that wrap around the side edge of the housing 10 (top housing 20 and bottom housing 30). The arms 72 can extend around at last a portion of the side and bottom surfaces of the housing 10 to engage the recessed surfaces 35 provided on the bottom surface 33 of the housing 10/bottom housing 30. The arms 72 can move along the recessed surfaces 35 between a first and second position. The ends of the recessed surfaces 35 can act as a stop for the slide cover 70.

The slide cover 70 can include an engagement feature for fixing the position of the slide cover 70 along the housing 10/top housing 20. For example, as illustrated in FIGS. 11B and 11E, the slide cover 70 can include recesses 73 on its bottom surface 74 for engaging a corresponding protrusion 25 provided in the top housing 20. The bottom surface 74 can also include an elongated channel 75 terminating proximate the circularshaped recess 73. It is contemplated that the engagement features, recess 73 and elongated channel 75, can have any suitable shape/profile corresponding to the protrusion 25. The recess 73 and elongated channel 75 can have a circular, elliptical, square, rectangular, or any other regular or irregular shape. As the slide cover 70 moves along the housing 10, the protrusions 25 provided in the top housing 20 move along the channel 75 of the slide cover 70. Once moved to the first or second position, the corresponding housing protrusion 25 engages the recess 73 provided in the slide cover 70. The surgeon can disengage the engagement features (protrusion/recess) using hand force.

The slide cover 70 can include a grip surface 76 to accommodate a secure grip and provide a leverage point to facilitate movement of the slide cover 70 by the surgeon. As illustrated in FIGS. 11A, 11C, 11D, the grip surface 76 can include a contoured protrusion extending from the top surface 77 of the slide cover 70. The grip surface 76 can also include a surface feature/texture on the top and/or side surfaces 77, 78 of the slide cover 70.

As outlined above, the slide cover 70 is slidably coupled to the housing 10 (top housing 20 and bottom housing 30). To provide a consistent profile along the control device 1, the side cover 70 can have a width and height corresponding to the width and height of the housing 1. As illustrated in FIGS. 1 and 2, the slide cover 70 can be received within the recessed surface 23 of the top housing 20 such that the width and height corresponds to the width and height of the housing 10. It is contemplated that the grip surface 76 of the slide cover 70 may extend above the width/height of the housing 10. However, in another example (not shown), the height/width of the grip surface of the slide cover 70 is less than the height/width of the housing 10.

During use of the control device 1, the control device 1 is first coupled to and positioned appropriately on the handpiece 110 of a surgical power tool 100. The surgeon may orient with control device 1 such that the bottom surface 33 of the housing 1 and the opening between arms 32 aligns with the handpiece 110. The control device 1 is then coupled to the handpiece 110 by a press-fit or snap-fit connection. For example, the arms 32 of the housing 10 can flex/separate to advance over and around the handpiece 110. Depending on the surgical procedure, patient anatomy, and surgeon grip position and visibility, the surgeon may wish to position the control device 1 at various locations along and/or around the handpiece 110 of the power tool 100. Accordingly, once coupled to the handpiece 110, the position of the control device 1 can be adjusted along/around the handpiece 110 as desired by the surgeon. For example, the surgeon can slide the control device 1 forward or rearward along the handpiece 110. The surgeon can also rotate the control device 1 around the perimeter of the handpiece 110.

If desired, the surgeon can fix the location of the control device 1 by engaging a mechanical fastener/connection between the housing 10 and the handpiece 110. Alternatively/in addition, the surgeon can fix the position of the control device 1 with respect to the handpiece 110 by grasping the control device 1 and handpiece 110 around the periphery. The surgeon can then move the slide cover 70 to an operating position such that the input surface 51 of the input element 50 is exposed. To move the slide cover 70, the surgeon may provide pressure on the grip surface 76 in the direction of desired movement sufficient to overcome engagement between the engagement features between the slide cover 70 and the top housing 10. In one example, lateral pressure may be applied to the protrusion extending from the top surface 77 of the slide cover 70. In another example, the surgeon may grasp the slide cover 76 at its side edges and pull/push the slide cover back, away from the input element 50.

With the control device 1 attached, the surgeon can then position the power tool 100 at its desired location at/in the surgical site to complete the medical procedure. Once positioned, the input element 50 can be activated by pressure applied by the surgeon's finger(s)/thumb at the input surface 51. In an example control device 1, depressing the input element 50 causes a carbon pill 55 overmolded into the input element 50 to complete an electrical connection with a corresponding contact 63 provided on the control unit 60. With the electrical connection in place, user input information is then transmitted to the power tool 100. By varying pressure on the input surface 51 the surgeon is able to vary the input information provided to the power tool 100. For example, increasing/decreasing the pressure provided on the input surface 51 can result in a corresponding increase/decrease in the speed, torque, etc. of the power tool 100. Once the medical procedure is complete (or the need for the power tool has ended), the surgeon can remove the power tool 100 from the surgical site. Before or after removing the power tool 100, the surgeon can reposition/close the slide cover 70 such that the slide cover 70 is positioned over the input surface 51. Closing the slide cover 70 is performed in a similar (but opposite) fashion as opening. The surgeon can provide pressure on the grip surface 76 in the direction of desired movement sufficient to overcome engagement between the engagement features between the slide cover 70 and the top housing 10. Once in the fully closed position, the recess 73 provided on the slide cover can engages the protrusion 25 provided in the top housing 10. By repositioning the slide cover 70 and engaging the protrusion 25, the surgeon prevents accidental activation of the power tool 100.

With the surgical tool 100 removed from the surgical site, the surgeon can then safely remove the control device 1 from the handpiece 110. If a mechanical fastener was used to fix the position on the handpiece 110 it must be disengaged. The surgeon can then slide/pull the control device 1 off of the handpiece 110. Once the control device is removed from the handpiece 110 it can be sterilized for reuse, recycled and/or disposed of. Because the control device 1 can be separated from the handpiece 110 (upon completion of or during the surgical procedure), the control device 1 can be easily be cleaned and sterilized separate from the power tool 100.

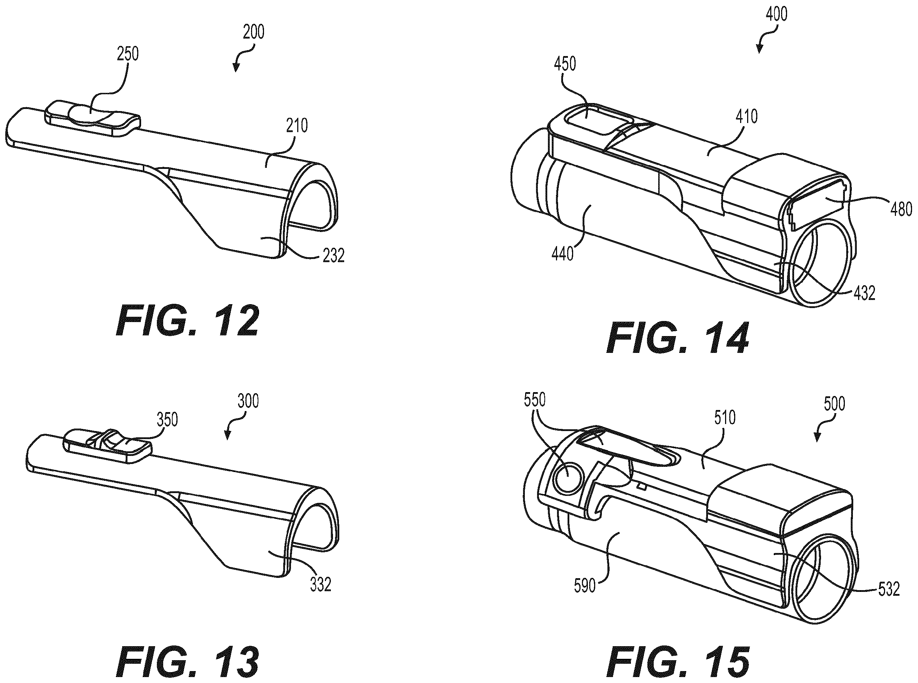

FIG. 12 provides another example control device 200. The control device 200 is similar to the control device 1 depicted in FIG. 1, like reference symbols are used to indicate like elements. The differences between the control device 200 and the control device 1 are discussed below. The control device 200 can include a sliding magnet 250 for providing input information to the control unit. As the magnet 250 is moved along the housing 210 is activates the contacts provided on the control unit. For example, the magnet 250 is moved between an on/off position along the housing 210, thereby providing on/off instructions to the power tool.

FIG. 13 provides another example control device 300. The control device 300 is similar to the control device 1 depicted in FIG. 1, like reference symbols are used to indicate like elements. The differences between the control device 300 and the control device 1 are discussed below. The control device 300 can also include a sliding magnet 350 for providing input information to the control unit. As the magnet 350 moves along the housing 310, the contacts on the control unit is activated. In the control device 300, the direction and/or location of the magnet 350 along the housing can be used vary the input information received at the control unit.

FIG. 14 provides another example control device 400. The control device 400 is similar to the control device 1 depicted in FIG. 1, like reference symbols are used to indicate like elements. The differences between the control device 400 and the control device 1 are discussed below. The control device 400 can include a popple/snap dome input element 450. Pressure provided at the input surface of the input element 450 completes the electrical connection between the input element 450 and contacts of the control unit. Releasing the pressure on the input surface disengages the electrical connection. As the popple/snap dome input element 450 is moved depressed/released a corresponding on/off instruction is sent to the power tool. In another example, the control device 400 includes a force sensor input element 450. As such, the varying the force/pressure applied at the input element 450 corresponds to a change in power tool operation. For example, as the pressure/force is increased the speed, torque, etc. of the power tool increases. The control device 400 can also include a replaceable and/or rechargeable battery 480. The battery 480 can be accessed via an opening provided at the proximal end of the control device 400.

FIG. 15 provides another example control device 500. The control device 500 is similar to the control device 1 depicted in FIG. 1, like reference symbols are used to indicate like elements. The differences between the control device 500 and the control device 1 are discussed below. The control device 500 can include a capacitive touch sensor input element 550. The capacitive input element 550 can include multiple input surfaces capable of providing different input information to the power tool. For example, the various input surface can each provide separate instructions regarding speed, torque, illumination, irrigation, or any other function completed by the power tool.

While the foregoing description and drawings represent the preferred embodiment of the present invention, it will be understood that various additions, modifications, combinations and/or substitutions may be made therein without departing from the scope of the present invention as defined the accompanying claims. In particular, it will be clear to those skilled in the art that the present invention may be embodied in other specific forms, structures, arrangements, proportions, and with other elements, materials, and components, without departing from the essential characteristics thereof as defined in the appended claims. One skilled in the art will appreciate that the invention may be used with many modifications of structure, arrangement, proportions, materials, and components and otherwise, used in the practice of the invention, which are particularly adapted to specific environments and operative requirements without departing from the scope of the present invention. In addition, features described herein may be used singularly or in combination with other features. The presently disclosed embodiments are, therefore, to be considered in all respects as illustrative and not restrictive, the scope of the invention being defined by the appended claims and not limited to the foregoing description.

It will be appreciated by those skilled in the art that changes could be made to the embodiments described above without departing from the broad concept thereof which is defined by the appended claims. It is understood, therefore, that this invention is not limited to the particular embodiments disclosed, but it is intended to cover modifications within the and scope of the present invention, as defined by the following claims.

## Claims

1. A control device for a surgical power tool comprising:
a housing (10) sized and configured to couple to a surgical power tool, the housing comprising a top housing (20) and a bottom housing (30), wherein the top housing and the bottom housing are coupled together;
an input element (50), the input element comprising a base portion (53) and an input surface (51) provided at the housing for receiving a user input, the input surface further comprising an anchor (54) which extends from the base portion (53) in a direction away from the input surface (51), the anchor configured to couple the input element to the bottom housing; and
a control unit (60) within the housing for sending user input information received at the input surface to the surgical power tool, the control unit comprising an opening (64),
where the anchor is configured to extend from the base portion of the input surface and through the opening provided in the control unit, to couple the input element to the bottom housing.

2. The device of claim 1, wherein the bottom housing (30) is sized and configured to couple to a surgical power tool.

3. The device of claim 2, wherein the top housing (20) is integrally formed with the bottom housing.

4. The device of claim 1, further comprising arms (72) extending from the housing (10), the arms sized and configured to engage at least a portion of an outer perimeter of a power tool, the arms extending from the housing in a direction away from a bottom surface of the housing and include an inner surface having a shape corresponding to an outer perimeter of a power tool.

5. The device of claim 4, wherein the arms (72) are sized and configured to couple the control device to a handpiece of a power tool, wherein the arms (72) couple the housing to a power tool by at least one of a snap fit, press fit, a mechanical fastener, a weld and an adhesive.

6. The device of claim 1, wherein the input surface is:
a) accessible via an opening in a top surface of the housing, input surface extending at least partially into the opening on the housing,
b) composed of a pressure responsive material, wherein the user input information is configured to vary in response to a pressure of the user input at the input surface, or
c) located proximate the control unit such that a user input at the input surface activates the control unit.

7. The device of claim 1, wherein the control unit includes a printed circuit board, a battery (80), and a wireless communication unit for sending electronic user input information to a surgical power tool (100), or the control unit is configured to receive operation information from a surgical power tool (100).

8. The device of claim 1, wherein the user input information is configured to vary in response to at least one of a location and a direction of the user input at the input surface.

9. The device of claim 1, further comprising a cover (70) slidably coupled to the housing such that the cover (70) is movable between a first position, over at least a portion of the input surface, and a second position, away from the input surface, the cover including a downwardly extending arm (72) slidably engaged to the housing.

10. A surgical tool and control device comprising:
a surgical power tool;
the control device according to claim 1, coupled to the surgical power tool, wherein:
the housing further comprises arms (72) for coupling the control device to the power tool, the arms extending from the housing and engaging at least a portion of the outer perimeter of a handpiece of the power tool;
the input surface comprises a pressure responsive touch pad located on a top surface of the control device;
the control unit is configured for sending user input information received at the touch pad to the power tool for directing a speed of the power tool;
wherein the control unit is movable along the handpiece of the surgical power tool.

11. The surgical tool and control device of claim 10, wherein the control unit includes a printed circuit board, a battery (80), and a wireless communication unit for sending user input information to the power tool,
wherein the user input information is received by a control unit (60) of the power tool and the speed of the power tool varies in response to a pressure of the user input received at the touch pad.

## Patentansprüche

1. Steuervorrichtung für ein chirurgisches Elektrowerkzeug, umfassend:
ein Gehäuse (10), das bemessen und konfiguriert ist, um an ein chirurgisches Elektrowerkzeug gekoppelt zu werden, wobei das Gehäuse ein oberes Gehäuse (20) und ein unteres Gehäuse (30) umfasst, wobei das obere Gehäuse und das untere Gehäuse miteinander gekoppelt sind;
ein Eingabeelement (50), wobei das Eingabeelement einen Basisabschnitt (53) und eine Eingabeoberfläche (51) umfasst, die am Gehäuse zum Empfangen einer Benutzereingabe bereitgestellt ist, wobei die Eingabeoberfläche ferner einen Anker (54) umfasst, der sich von dem Basisabschnitt (53) in einer Richtung weg von der Eingabeoberfläche (51) erstreckt, wobei der Anker konfiguriert ist, um das Eingabeelement mit dem unteren Gehäuse zu koppeln; und
eine Steuereinheit (60) innerhalb des Gehäuses zum Senden von an der Eingabeoberfläche empfangenen Benutzereingabeinformationen an das chirurgische Elektrowerkzeug, wobei die Steuereinheit eine Öffnung (64) umfasst, wobei der Anker konfiguriert ist, um sich von dem Basisabschnitt der Eingabeoberfläche und durch die in der Steuereinheit bereitgestellte Öffnung zu erstrecken, um das Eingabeelement mit dem unteren Gehäuse zu koppeln.

2. Vorrichtung nach Anspruch 1, wobei das untere Gehäuse (30) bemessen und konfiguriert ist, um mit einem chirurgischen Elektrowerkzeug gekoppelt zu werden.

3. Vorrichtung nach Anspruch 2, wobei das obere Gehäuse (20) einstückig mit dem unteren Gehäuse ausgebildet ist.

4. Vorrichtung nach Anspruch 1, ferner umfassend Arme (72), die sich von dem Gehäuse (10) erstrecken, wobei die Arme bemessen und konfiguriert sind, um mit mindestens einem Abschnitt eines Außenumfangs eines Elektrowerkzeugs in Eingriff zu kommen, wobei sich die Arme von dem Gehäuse in einer Richtung weg von einer unteren Oberfläche des Gehäuses erstrecken und eine innere Oberfläche einschließen, die eine Form aufweist, die einem Außenumfang eines Elektrowerkzeugs entspricht.

5. Vorrichtung nach Anspruch 4, wobei die Arme (72) bemessen und konfiguriert sind, um die Steuervorrichtung mit einem Handstück eines Elektrowerkzeugs zu koppeln, wobei die Arme (72) das Gehäuse mit einem Elektrowerkzeug durch mindestens eines von einer Schnappverbindung, einer Presspassung, einem mechanischen Befestigungselement, einer Schweißung und einem Klebstoff koppeln.

6. Vorrichtung nach Anspruch 1, wobei die Eingabefläche:
a) zugänglich über eine Öffnung in einer oberen Oberfläche des Gehäuses ist, wobei sich die Eingabeoberfläche mindestens teilweise in die Öffnung am Gehäuse erstreckt,
b) aus einem druckempfindlichen Material besteht, wobei die Benutzereingabeinformationen konfiguriert sind, um als Reaktion auf einen Druck der Benutzereingabe an der Eingabeoberfläche zu variieren, oder
c) nahe der Steuereinheit angeordnet ist, sodass eine Benutzereingabe an der Eingabeoberfläche die Steuereinheit aktiviert.

7. Vorrichtung nach Anspruch 1, wobei die Steuereinheit eine Leiterplatte, eine Batterie (80) und eine drahtlose Kommunikationseinheit zum Senden von elektronischen Benutzereingabeinformationen an ein chirurgisches Elektrowerkzeug (100) einschließt, oder die Steuereinheit konfiguriert ist, um Betriebsinformationen von einem chirurgischen Elektrowerkzeug (100) zu empfangen.

8. Vorrichtung nach Anspruch 1, wobei die Benutzereingabeinformationen konfiguriert ist, um als Reaktion auf mindestens eine von einer Position und einer Richtung der Benutzereingabe an der Eingabeoberfläche zu variieren.

9. Vorrichtung nach Anspruch 1, ferner umfassend eine Abdeckung (70), die verschiebbar an das Gehäuse gekoppelt ist, sodass die Abdeckung (70) zwischen einer ersten Position über mindestens einem Abschnitt der Eingabeoberfläche und einer zweiten Position weg von der Eingabeoberfläche bewegbar ist, wobei die Abdeckung einen sich nach unten erstreckenden Arm (72) einschließt, der verschiebbar mit dem Gehäuse in Eingriff steht.

10. Chirurgisches Werkzeug und Steuervorrichtung, umfassend:
ein chirurgisches Elektrowerkzeug;
die Steuervorrichtung nach Anspruch 1, gekoppelt an das chirurgische Elektrowerkzeug, wobei:
das Gehäuse ferner Arme (72) zum Koppeln der Steuervorrichtung mit dem Elektrowerkzeug umfasst, wobei sich die Arme von dem Gehäuse erstrecken und mit mindestens einem Abschnitt des Außenumfangs eines Handstücks des Elektrowerkzeugs in Eingriff stehen;
die Eingabeoberfläche ein druckempfindliches Touchpad umfasst, das auf einer oberen Oberfläche der Steuervorrichtung angeordnet ist;
die Steuereinheit konfiguriert ist, um am Touchpad empfangene Benutzereingabeinformationen an das Elektrowerkzeug zu senden, um eine Geschwindigkeit des Elektrowerkzeugs zu steuern;
wobei die Steuereinheit entlang des Handstücks des chirurgischen Elektrowerkzeugs bewegbar ist.

11. Chirurgische Werkzeug und Steuervorrichtung nach Anspruch 10, wobei die Steuereinheit eine Leiterplatte, eine Batterie (80) und eine drahtlose Kommunikationseinheit zum Senden von Benutzereingabeinformationen an das Elektrowerkzeug einschließt,
wobei die Benutzereingabeinformationen von einer Steuereinheit (60) des Elektrowerkzeugs empfangen wird und die Geschwindigkeit des Elektrowerkzeugs als Reaktion auf einen Druck der an dem Touchpad empfangenen Benutzereingabe variiert.

## Revendications

1. Dispositif de commande pour un outil électrique chirurgical comprenant :
un boîtier (10) dimensionné et conçu pour être accouplé à un outil électrique chirurgical, le boîtier comprenant un boîtier supérieur (20) et un boîtier inférieur (30), dans lequel le boîtier supérieur et le boîtier inférieur sont accouplés ensemble ;
un élément d'entrée (50), l'élément d'entrée comprenant une partie base (53) et une surface d'entrée (51) prévue au niveau du boîtier pour recevoir une entrée utilisateur, la surface d'entrée comprenant en outre un ancrage (54) qui s'étend à partir de la partie base (53) dans une direction s'éloignant de la surface d'entrée (51), l'ancrage étant conçu pour accoupler l'élément d'entrée au boîtier inférieur ; et
une unité de commande (60) à l'intérieur du boîtier pour envoyer des informations d'entrée utilisateur reçues au niveau de la surface d'entrée à l'outil électrique chirurgical, l'unité de commande comprenant une ouverture (64),
où l'ancrage est conçu pour s'étendre à partir de la partie base de la surface d'entrée et à travers l'ouverture prévue dans l'unité de commande, pour accoupler l'élément d'entrée au boîtier inférieur.

2. Dispositif selon la revendication 1, dans lequel le boîtier inférieur (30) est dimensionné et conçu pour être accouplé à un outil électrique chirurgical.

3. Dispositif selon la revendication 2, dans lequel le boîtier supérieur (20) est formé solidairement avec le boîtier inférieur.

4. Dispositif selon la revendication 1, comprenant en outre des bras (72) s'étendant à partir du boîtier (10), les bras étant dimensionnés et conçus pour venir en prise avec au moins une partie d'un périmètre externe d'un outil électrique, les bras s'étendant à partir du boîtier dans une direction s'éloignant d'une surface inférieure du boîtier et comportant une surface interne ayant une forme correspondant à un périmètre externe d'un outil électrique.

5. Dispositif selon la revendication 4, dans lequel les bras (72) sont dimensionnés et conçus pour accoupler le dispositif de commande à une pièce à main d'un outil électrique, dans lequel les bras (72) accouplent le boîtier à un outil électrique au moyen d'au moins l'un parmi un encliquetage, un ajustement serré, une attache mécanique, une soudure et un adhésif.

6. Dispositif selon la revendication 1, dans lequel la surface d'entrée est :
a) accessible par l'intermédiaire d'une ouverture dans une surface supérieure du boîtier, la surface d'entrée s'étendant au moins partiellement dans l'ouverture sur le boîtier,
b) constituée d'un matériau sensible à la pression, dans lequel les informations d'entrée utilisateur sont configurées pour varier en réponse à une pression de l'entrée utilisateur au niveau de la surface d'entrée, ou
c) située à proximité de l'unité de commande de telle sorte qu'une entrée utilisateur au niveau de la surface d'entrée active l'unité de commande.

7. Dispositif selon la revendication 1, dans lequel l'unité de commande comporte une carte de circuit imprimé, une batterie (80) et une unité de communication sans fil pour envoyer des informations électroniques d'entrée utilisateur à un outil électrique chirurgical (100), ou l'unité de commande est configurée pour recevoir des informations de fonctionnement provenant d'un outil électrique chirurgical (100).

8. Dispositif selon la revendication 1, dans lequel les informations d'entrée utilisateur sont configurées pour varier en réponse à au moins l'un parmi un emplacement et une direction de l'entrée utilisateur au niveau de la surface d'entrée.

9. Dispositif selon la revendication 1, comprenant en outre un élément de protection (70) accouplé de manière coulissante au boîtier de telle sorte que l'élément de protection (70) est mobile entre une première position, sur au moins une partie de la surface d'entrée, et une seconde position, à l'écart de la surface d'entrée, l'élément de protection comportant un bras (72) s'étendant vers le bas et venant en prise de manière coulissante avec le boîtier.

10. Outil chirurgical et dispositif de commande comprenant :
un outil électrique chirurgical ;
le dispositif de commande selon la revendication 1, couplé à l'outil électrique chirurgical, dans lequel :
le boîtier comprend en outre des bras (72) pour coupler le dispositif de commande à l'outil électrique, les bras s'étendant à partir du boîtier et venant en prise avec au moins une partie du périmètre externe d'une pièce à main de l'outil électrique ;
la surface d'entrée comprend un pavé tactile sensible à la pression situé sur une surface supérieure du dispositif de commande ;
l'unité de commande est configurée pour envoyer des informations d'entrée utilisateur reçues au niveau du pavé tactile à l'outil électrique pour régler une vitesse de l'outil électrique ;
dans lequel l'unité de commande est mobile le long de la pièce à main de l'outil électrique chirurgical.

11. Outil chirurgical et dispositif de commande selon la revendication 10, dans lequel l'unité de commande compote une carte de circuit imprimé, une batterie (80), et une unité de communication sans fil pour envoyer des informations d'entrée utilisateur à l'outil électrique,
dans lequel les informations d'entrée utilisateur sont reçues par une unité de commande (60) de l'outil électrique et la vitesse de l'outil électrique varie en réponse à une pression de l'entrée utilisateur reçue au niveau du pavé tactile.
